# EUROPEAN PATENT APPLICATION

(11) **EP 2 491 952 A1**
(43) Date of publication of application: **29.08.2012**
(21) Application number: 11155275.8
(22) Date of filing: 22.02.2011
(51) Int. Cl.: A61K 47/48, C12N 15/87

(54) **A system for cargo delivery into the cells**

(71) Applicant: Cepep III AB, 104 30 Stockholm (SE)
(72) Inventor: Langel, Ülo, 51013 Tartu (EE); Oskolkov, Nikita, 50705 Tartu (EE); Arukuusk, Piret, 51006 Tartu (EE); Copolovici, Dana Maria, 420146 Bistrita (RO)
(74) Representative: Sarap, Margus

(57) **Abstract**

The present invention relates to a system for intracellular cargo delivery, named NickFect, comprising at least one component A, which is attached covalently to cell penetrating peptide B and/or peptide or non-peptide construct C. The said delivery system NickFect relates to chemically modified new cell-penetrating peptides (CPP) non-covalently or covalently complexed with cargo for efficient cellular.

## Description

### FIELD OF THE INVENTION

The present invention relates to a system for intracellular cargo delivery, named NickFect, comprising at least one component A, which is attached covalently to cell penetrating peptide B and/or peptide or non-peptide construct C. The said delivery system NickFect relates to chemically modified new cell-penetrating peptides (CPP) non-covalently or covalently complexed with cargo for efficient cellular. More precisely, the invention relates to constructs comprising non-toxic peptide delivery vector, with increased stability and more potent to escape from endosomal-lysosomal compartment and is capable to form stable complexes with cargo, which remain intact in the presence of serum.

The invention relates on a delivery system, which has overall negative charge of formed nanoparticles for oligonucleotide delivery.

The invention also relates on a delivery system, which comprises branched CPP, where fatty acid modified TP10 or its segment are linked through α,β,γ,ε - amino groups of lysine or their analogues to amphipatic or/and α-helical peptide or peptide segments.

Further, the invention relates to a method of delivering cargos (RNA, DNA, drugs, plasmids, minicircles etc.) into cytosol or nucleus of a target cell *in vitro* or *in vivo.* It also relates to the use of the system in diagnosis of deceases, as research tool and as a targeting system, a composition comprising the system and especially a pharmaceutical composition a material covered with the system and a material having the delivery systems into material. Finally also relates to novel cell-penetrating peptides.

### BACKGROUND OF THE INVENTION

The hydrophobic cell membrane is a lipid bilayer that encloses the cellular contents. It functions as barrier that normally only small and/or hydrophobic molecules can cross and prevent access to the interior of cells of other macromolecules like naked DNA, RNA or proteins. Although few strategies were designed to solve this problem in last decade, the inability to cross the plasma membrane is still one of the major hindrance to overcome in order to success current drug research and development (R&D).

During the past 40 years, several oligonucleotide (ON)-based methods have been developed with the purpose of regulating gene expression. The efficiency of this methods depends, among the other factors, on the efficient uptake of ON into the cells and subsequent endosomal escape. The other most basic method for gene regulation involves the use of bacterial vectors to express the genes of interest. In addition to evaluate functional aspects of different genes, this is a highly appealing strategy to utilize in clinical settings, i.e. gene therapy. Gene therapy was originally thought to serve as corrective treatment for inherited genetic diseases, for example, by splice-correcting oligonucleotides. However, over the past 15 years, experimental gene therapy for cancer diseases has become the most frequent application although other acquired diseases have also been investigated [1].

Other versatile strategies utilizing shorter ON-sequences to interfere with gene expression have emerged. Antisense approaches based on double-stranded RNA molecules, 20-25 nucleotides in length, interfering RNAs (siRNAs) that are utilized to confer gene silencing or splice correcting ONs (SCOs), applied for the manipulation of splicing patterns, have been rigorously exploited recently [2,3]. Although being efficient biomolecule for regulating gene expression, their hydrophilic nature and size (in case of plasmid over one MDa in size) prohibits cellular internalization.

Thus, in order to progress current gene therapy, various cellular delivery systems are required to transport different molecules into the cell, but most of them have some drawbacks, like high cytotoxity, inherent immunogenicity, low transfection efficiency, etc **(Table 1).**

**Table 1: Delivery vectors**

| **Transfection reagent** | **Drawbacks** |
|---|---|
| Viral vectors (adenoviral, recombinant lentiviral vectors, etc.) | Endogenous recombination, oncogenic effects and immunological reactions |
| Cationic liposomes (Lipofectamine™ 2000 - a widely used transfection reagent, produced and sold by Invitrogen) | High cytotoxicity, not usable *in vivo,* unable to transfect the entire cell population |
| Peptide-based reagents (Transductin™ - is a delivery reagent for research applications, developed by Dr. Steven Dowdy (UCSD School of Medicine & Traversa Therapeutics, Inc)) | Serum-sensitive, for siRNA transfection bigger amount of biomolecules are required, mainly used for siRNAs transfection |
| Cationic polymers | Non-biodegradable, low solubility, high cytotoxicity |
| Physical methods(eg. electroporation) | Cell damage and non-specific transport |
| Lipoplex | Low efficiency and poor reproducibility of delivery |

Therefore, cell-penetrating peptides have great potential and promise *as in vitro* and *in vivo* delivery vectors, so they can be used in research and medicine fields. Cell-penetrating peptides (CPPs), also called protein transduction domains (PTDs) are a class of peptides that has drawn much attention in the last decades as non-toxic vehicles for delivery of wide range of cargos. These peptides are usually less than 30 amino acids (aa) in length with net positive charge and/or amphipathic nature and are able to deliver payload over cell membranes both *in vitro* and *in vivo* [5]. Today there are hundreds of known CPPs. In order to promote intracellular delivery CPPs should be conjugated with cargo (oligonucleotide, plasmid, minicicle etc.) in covalent or non-covalent manner. Covalent attachment of peptide to ON or any other biomolecule is laborious procedure and usually high concentrations of peptide conjugates are needed to obtain a significant biological response. In case of non-covalent complexes, the efficient concentrations are in nanomolar range and non-covalent complex formation can be reached by mixing together peptide and biomolecule solutions in water for 1 hour.

### SUMMARY OF THE INVENTION

The present invention provides a system for intracellular cargo delivery comprising a new series of cell-penetrating peptides that overcome the drawbacks of non-covalent gene-delivery, low and heterogeneous delivery as well as toxicity.

### BRIEF DESCRIPTION OF THE DRAWINGS

**Figure 1A**. Pre-mRNA of the modified luciferase gene. The intron 2 from the B-globin gene carrying a point mutation at nucleotide 705 is inserted into the luciferase gene. Blockage of this site with SCO redirects splicing towards the functional mRNA. **Fig 1B**, **1C**. General schematic structures of NickFect delivery system, where solid line marked for covalent binding and dotted line is non-covalent conjugation.
**Figure 2****.** Splice correction after treatment with phosphorylated CPPs (NF1, NF5) or Stearyl-TP10 and Lipofectamine 2000 complexed with 200 nM SCO.
   Hela pLuc 705 cells were treated with peptide:2'-OMe ON complexes at different molar ratios (5:1, 7:1, 10:1) in serum free DMEM for 4 hours, after which media was replaced for full growth media and incubated additionally 20 hours. Lipofectamine 2000 was used according to manufactures protocol. The results clearly show that in addition to phosporylation modification in the backbone of the peptide is necessary to increase splice correction efficacy.

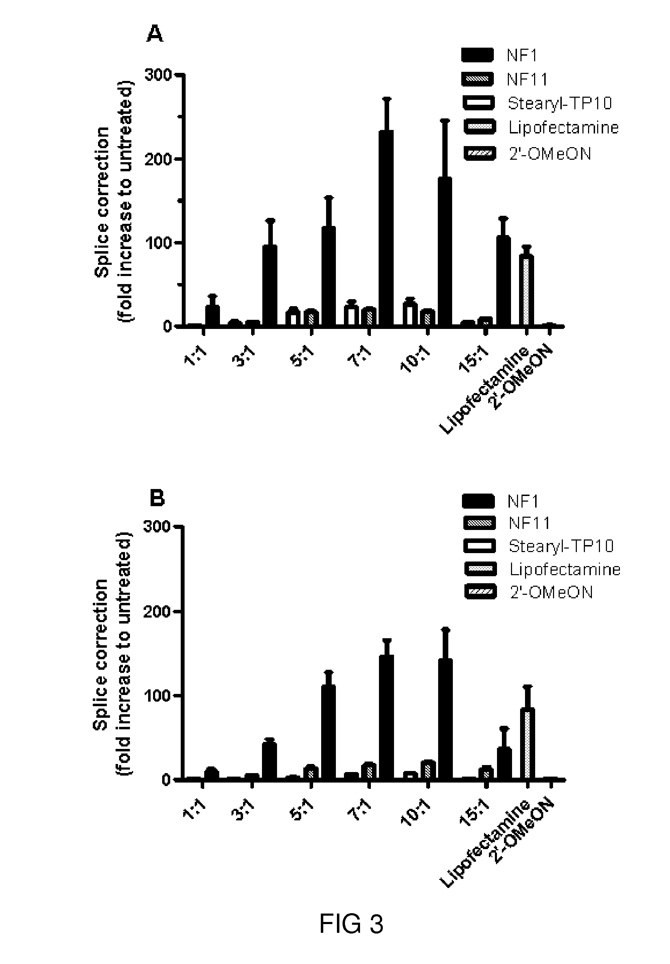

**Figure 3****.** NickFects are efficient transfection vectors at low peptide:ON molarratios. The complexes were applied at molar ratios 1:1, 3:1, 5:1, 7:1, 10:1, 15:1 to Hela pLuc 705 cells in SFM - A and in FM - B, at 200 nM ON concentration. Lipofectamine 2000 was used according to manufactures protocol. NickFect1 induced a dramatic increase in splice correction and very low amounts of peptides are needed to gain a biological response.

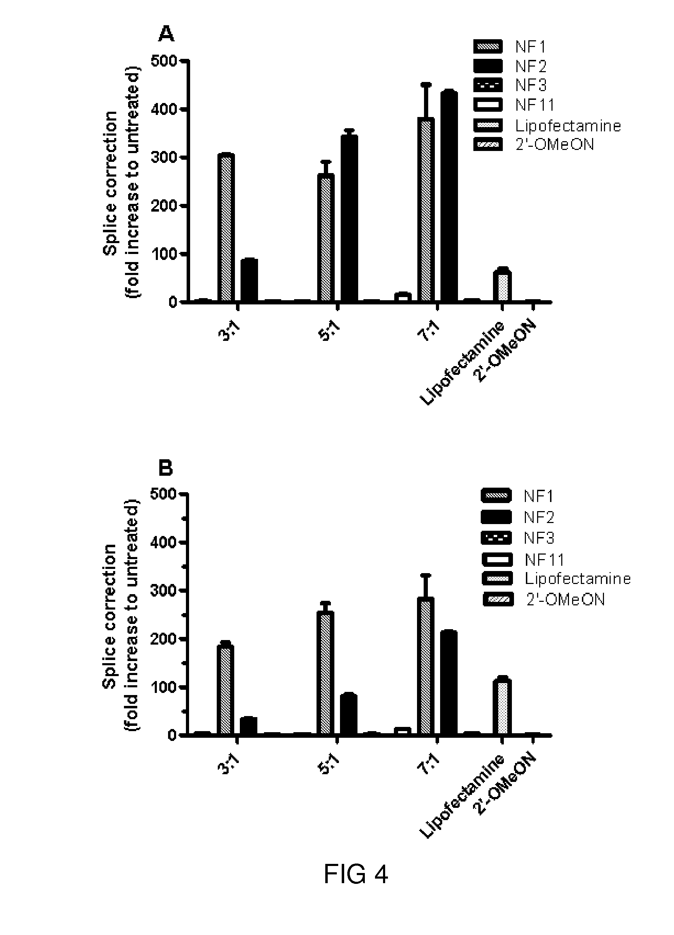

**Figure 4****.** Effect of the introduction of phosphoryl groups at tyrosine or threonine moieties in chemically modified Stearyl-TP10 analog (NF11) on the splice correction activity. The complexes were applied at molar ratios 3:1, 5:1, 7:1 to Hela pLuc 705 cells in SFM - A and in FM - B, at 200 nM ON concentration. Lipofectamine 2000 was used according to manufactures protocol. New phosphorylated CPPs induced a dramatic increase in splice correction and very low amounts of peptides and ONs are needed to gain a biological response. NF1 acted more effectively in full media, compared to NF2.

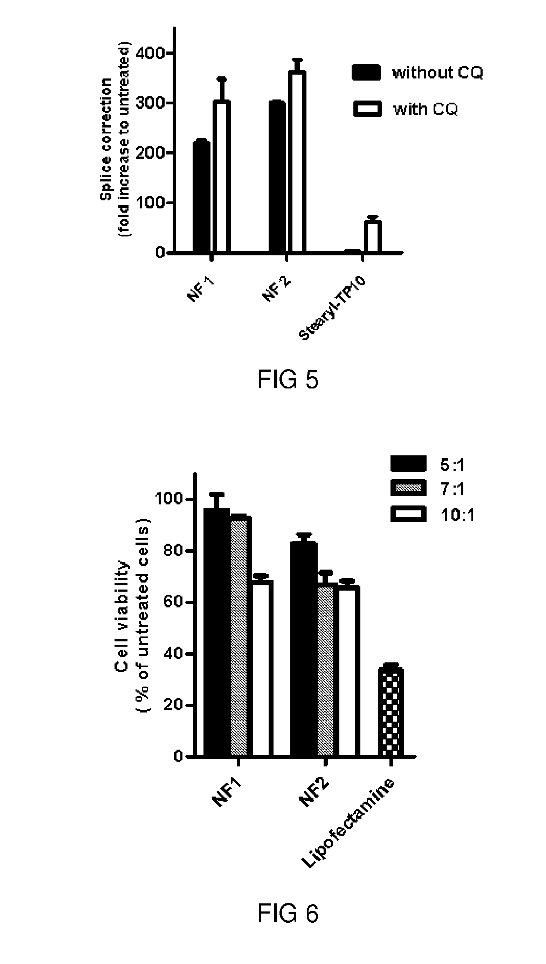

**Figure 5****.** The effect of the lysosomotropic reagent chloroquine on splice-correction activity. The peptide-ON complex NF1, NF2 and Stearyl-TP10 in SFM were applied to the cells at most efficient molar ratio (7:1) at 200 nM ON concentration with and without the presence of chloroquine.
**Figure 6****.** The effect of oligonucleotide-peptide complexes on the viability of Hela cells as compared to Lipofectamine^{™} 2000. The cell viability was measured by MTS assay after adding of peptide:2'-OMe ON complexes at different molar ratios after 24 hours. ON applied at was 200 nM concentration. NF1 and NF2 were not toxic at most effective concentrations.

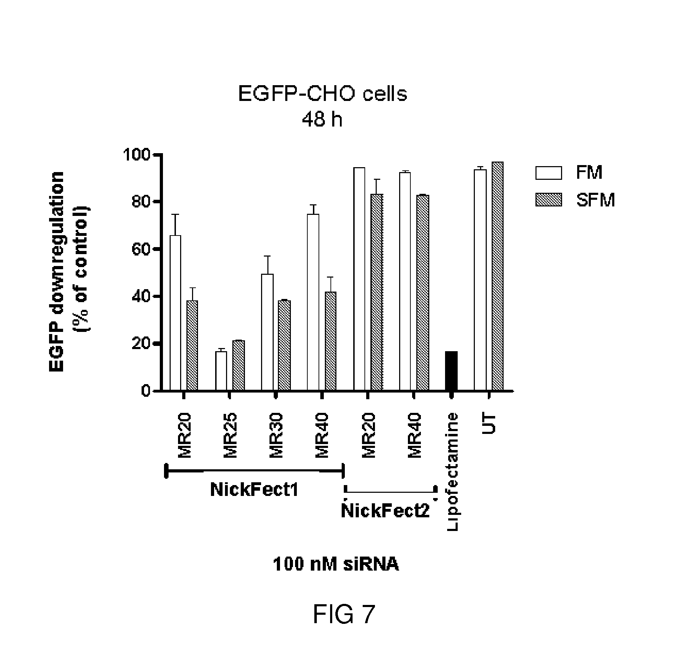

**Figure 7****.** NF1, NF2 mediated siRNA delivery into EGFP-CHO cells, which are stably expressing GFP protein. Cells were treated with siRNA:pepteide complexes at MR 20, MR25, MR30, MR40 in serum free DMEM media for 4 hours and with HAM full media for 20 hours. After that cells were washed, trypsinized and analysed by flow cytometry.

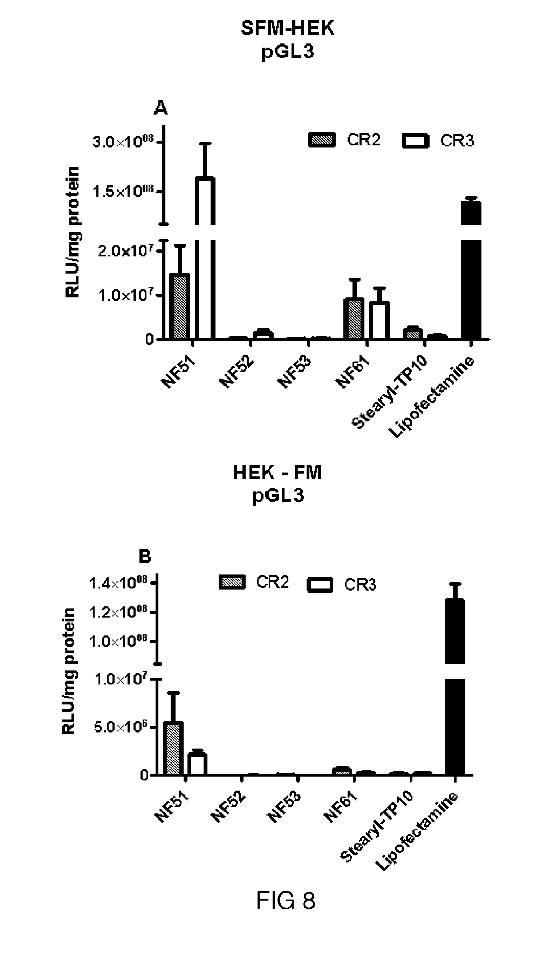

**Figure 8****.** NF51, NF52, NF53 and NF61 as delivery vector for pGL3 plasmid transfection in HEK293 cells.

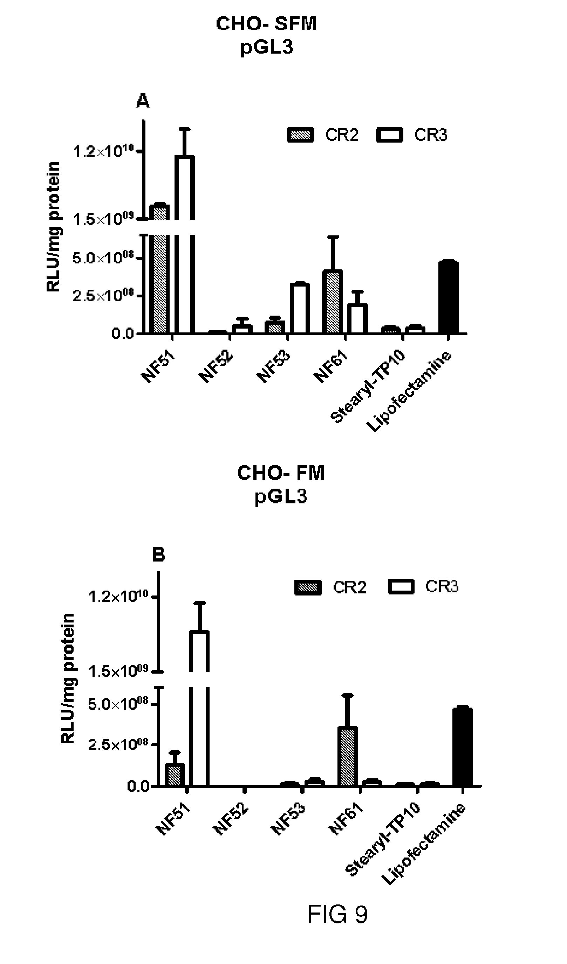

**Figure 9****.** NF51, NF52, NF53 and NF61 as delivery vector for pGL3 plasmid transfection in CHO cells.

### DETAILED DESCRIPTION OF THE INVENTION

The present invention provides the series of new chemically modified delivery vectors, NickFects, that are based on TP10 sequence (A **G Y L L G K I N L K A L A A L A K K I L-NH₂**) and that can be utilized for efficient delivery of different cargos (RNA, DNA, plasmid, minicircle, drugs, etc.) into cytosol and/or nucleus of a target cell *in vitro* or *in vivo* using a non-covalent or covalent approach. The new delivery system, NickFect, is able to form stable, tightly packed, regular and spherical nanoparticles with cargo. Formed nanoparticles have better interaction properties with plasma and/or nucleus membrane and can more efficiently internalizes into cells.

The abovementioned NickFects can be used in diagnosis of diseases, in gene therapy, in tumor treatment and for other pharmaceutical applications, as a research tool and as a targeting system.

More precisely, the invention relates to constructs comprising non-toxic, with increased stability and more potent to escape from endosomal-lysosomal compartment and which are capable to form stable complexes with cargo, which remain intact in the presence of serum NickFect delivery vectors are more efficient than the commercial transfection reagent Lipofectamine™2000 in conveying various ONs and plasmids inside cells, while being less toxic. The low toxicity of NickFects renders them suitable for transfection in sensitive cell systems and *in vivo,* while Lipofectamine™2000 can be used only in vitro due to its high toxicity at concentrations needed.

NickFects transfect 100% of cell population, while Lipofectamine transfects after optimization process only 60-80%, depending on the cell line.

NickFects can be stored as a powder for a long period of time, while the producer guarantees the stability of Lipofectamine™2000 for only 6 months at +4 °C.

Furthermore, the NickFect delivery vectors are more potent than conventionally used CPPs for RNA and DNA cellular delivery. Most important, only very low amounts of peptides and ONs are needed to gain a biological response.

NickFects are not only active for transportation of ON compounds acting in the nucleus of cells but are efficiently utilized for the delivery of cytoplasmically active ONs such as siRNAs. NF1 is as active as Lipofectamine 2000 for the delivery of siRNAs into cells. While Lipofectamine/siRNA complexes rarely generates more than 80% down-regulation of gene expression at any given siRNA concentration, NickFect1 complexed with siRNA confers almost complete RNAi at low siRNA concentrations.

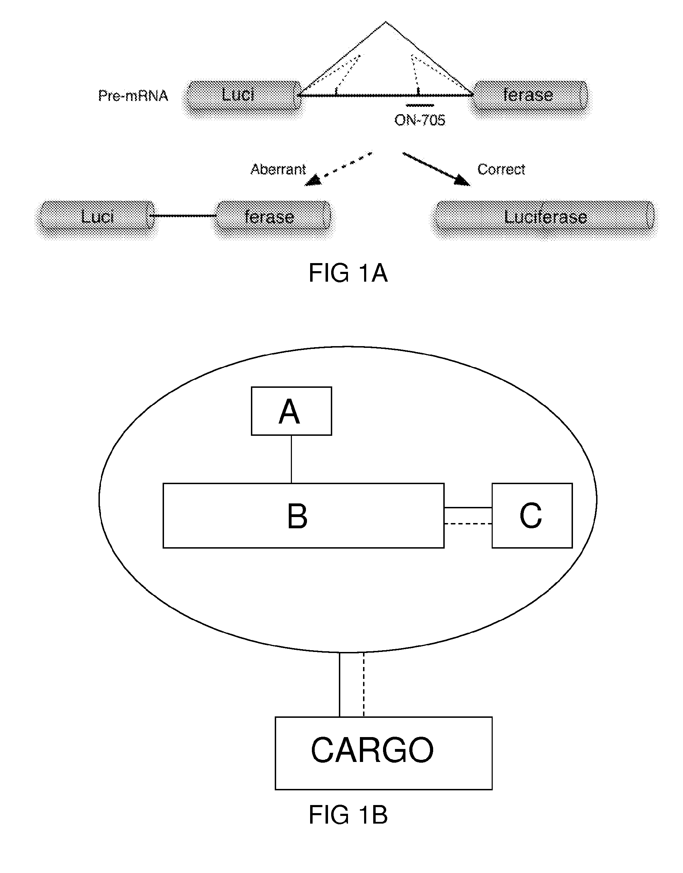

The present invention relates to a system for intracellular cargo delivery, named NickFect, comprising at least one cell penetrating peptide to B, which in some occasions comprises covalently linked component A and/or peptide or non-peptide construct C, which is targeting moiety. The said delivery system NickFect is capable of delivering cargo by covalent or non-covalent attachment (**Fig. 1B**)

The cell delivery system may comprise more than one peptide B, more than one component A and more than one targeting component C coupled to each other in any order without any cargo.

The abovementioned delivery system may be linked to one or more cargoes which may be delivered into cells, tissues or across a cell layer.

The invention also relates to novel cell-penetrating peptides as well as the method how to produce the NickFect constructs.

The component C as a targeting moiety is capable of reaching specific cells or tissues of interest. The targeting moiety may be an aptamer or targeting peptide such as a homing peptide or a receptor ligand.

It has been shown that the replacement of natural amino acid with non-natural amino acid (e.g. Ornithine) and change its secondary structure by using non α-amino group for peptide bond formation stabilize the CPP and make NickFects very potent vectors for efficient intracellular delivery of plasmids.

### Component A.

Component A comprises phosphate group (PO₃) or any negatively charged moiety (Asp, Glu, carbohydrates, etc.). Component A can be even a peptide sequence with overall negative charge. One linear aliphatic component A may be conjugated to peptide B or two similar or different components A may be conjugated to peptide B via lysine branched spacer.

Insertion of one or several negatively charged moieties a new path, as cell penetrating peptides are known to have positive net charge.

### Peptide B.

Peptide B comprises chemical modifications of cell penetrating peptide TP10 which has any fatty acid (e.g. stearic acid) covalently linked to the peptide backbone.

Peptide B also comprises cell penetrating peptide, fatty acid modified TP10, where in position 3, Tyr is replaced with Lys, Orn for subsequent addition of negatively charged molecules or/groups, with Thr, Ser for usage as tyrosine analogs and with Asp or Glu for insertion of carboxyl groups in peptide backbone.

Peptide B also comprises cell penetrating peptide, fatty acid modified TP10, where in position 8, Ile is replaced with Thr, Ser, Tyr, Asp or Glu any other hydrophilic amino acid to increase hydrophilic properties α-helix.

Peptide B may also be fatty acid modified TP10, where at least one Leucine is replaced with Leucine isomers and/or analogues (e.g. Norleucine).

Peptide B may also be fatty acid modified TP10, where at least one Lysine is replaced with Lysine isomers and/or derivatives or Ornitine isomers or/and analogues.

Replacement of natural amino acids by non- natural amino acids makes peptide more stable to serum proteases.

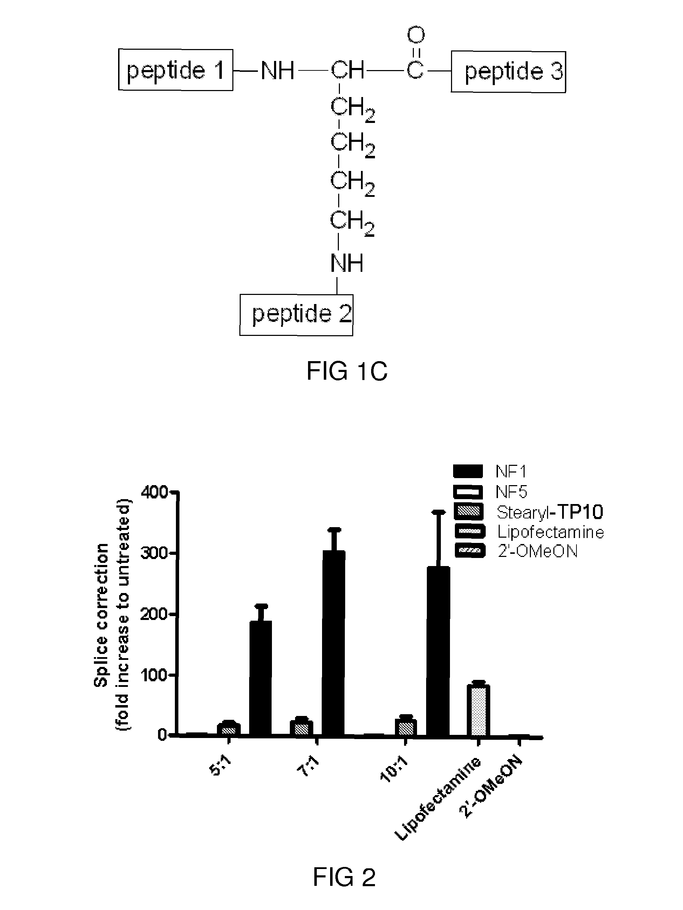

Peptide B may be a branched peptide comprising of fatty acid modified TP10 peptide or/and its segments (e.g. Galanin, Mastoparan) linked through α, β, γ, δ, ε-amino groups of lysine or their analogues to amphipatic or/and α-helical peptides or/and peptide segments (e.g. NPY, substance P, bradykinin, model sequences like (Ala-Leu)" , TP10, Galanin, Mastoparan) (**Fig. 1C**)

Several of these modifications can be made simultaneously.

### Construct C

Peptide or non-peptide construct C comprises targeting component capable of reaching specific cells or tissues of interest and which is covalently or non-covalently linked with peptide B.

Construct C is a cell- or tumor homing peptide, aptamer, a receptor ligand, a spacer comprising a cleavable site coupled to an inactivating peptide, peptide ligand, cytotoxic peptide, bioactive peptide ligand for a known or unknown receptor, a peptide sequence which selectively binds to a certain tissue or cell type or nuclear localization sequence (NLS).

### Spacer

Spacers may be used for the attachment of component A, C and cargoes to the component B.

The spacer may be a linear or branched moiety comprising of one or several Lysine and/or Ornithine residues.

### The Cargo

Cargo is attached to the delivery system by covalent assembly or complex formation.

The cargo may be selected from the group consisting of oligonucleotides and modified versions thereof, single-stranded oligonucleotides (DNA, RNA, PNA, LNA and synthetic oligonucleotides), double-strand oligonucleotides (siRNA, shRNA, decoy dsDNA etc), plasmids, minicircles and other varieties thereof, synthetic nucleotide analogs for the purpose of inhibition of viral replication or antiviral ONs.

The cargo may be a detection marker imaging agent, labeling molecule, a fluorescent marker , aptamer, a receptor ligand, a spacer comprising a cleavable site coupled to an inactivating peptide, peptide ligand, cytotoxic peptide, bioactive peptide, antibody, diagnostic agent, protein, pharmaceutical e.g. anticancer drug or antibiotics.

The anticancer drugs as a cargo may be chosen from an alkylating agent, an antimetabolite and a cytotoxic antibiotic.

In a further aspect of the invention of the constructs according to the invention may be used in diagnosis of diseases, in gene therapy, as research tool, as targeting system and as pharmaceutical composition.

The invention will now be further illustrated by short description of the drawings, materials and methods, examples including figures and figure legends as well as sample sequences, but it should be understood that the scope of the invention is not limited to any specifically mentioned embodiments or details.

**Tabel 2. Sample sequences of NickFects (NF-s)**

| Name | Sequence |
|---|---|
| NickFect 1 | Stearyl- AG**Y**(PO₃)LLGK**T**NLKALAALAKKIL-NH₂ |
| NickFect 2 | Stearyl- AGYLLGK**T**(PO₃)NLKALAALAKKIL-NH₂ |
| NickFect 3 | Stearyl- AG**Y**(PO₃)LLGK**T**(PO₃)NLKALAALAKKIL-NH₂ |
| NickFect 4 | AG**Y**(PO₃)LLGK(Stearyl)**T**NLKALAALAKKIL-NH₂ |
| NickFect 5 | Stearyl- AG**Y**(PO₃)LLGK**I**NLKALAALAKKIL-NH₂ |
| NickFect 6 | Stearyl- AG**K**(K-2PO₃)LLGK**I**NLKALAALAKKIL-NH₂ |
| NickFect 7 | AG**K**(K-2PO₃)LLGK(Stearyl)**I**NLKALAALAKKIL-NH₂ |
| NickFect 11 | Stearyl- AGYLLGK**T**NLKALAALAKKIL-NH₂ |
| NickFect 12 | Stearyl- AGYLLG**KS**NLKALAALAKKIL-NH₂ |
| NickFect 13 | Stearyl- AGYLLGK**Y**NLKALAALAKKIL-NH₂ |
| NickFect 14 | Stearyl- AG**D**LLGK**I**NLKALAALAKKIL-NH₂ |
| NickFect 15 | Stearyl- AG**E**LLGK**I**NLKALAALAKKIL-NH₂ |
| NickFect 21 | AGYLLGK(Stearyl)**T**NLKALAALAKKIL-NH₂ |
| NickFect 22 | AGYLLGK(Stearyl)**S**NLKALAALAKKIL-NH₂ |
| NickFect 23 | AGYLLGK(Stearyl)**Y**NLKALAALAKKIL-NH₂ |
| NickFect 24 | AG**D**LLGK(Stearyl)**I**NLKALAALAKKIL-NH₂ |
| NickFect 25 | AG**E**LLGK(Stearyl)**I**NLKALAALAKKIL-NH₂ |
| NickFect 31 | Stearyl- AG**K**(K-Glu₂)LLGK**I**NLKALAALAKKIL-NH₂ |
| NickFect 32 | Stearyl- AG**K**(K-Asp₂)LLGK**I**NLKALAALAKKIL-NH₂ |
| NickFect 33 | Stearyl- AG**K**(K-Asp, Glu)LLGK**I**NLKALAALAKKIL-NH₂ |
| NickFect 34 | Stearyl- AG**Y**LLGK(K-Glu₂)**I**NLKALAALAKKIL-NH₂ |
| NickFect 35 | Stearyl- AG**Y**LLGK(K-Asp₂)**I**NLKALAALAKKIL-NH₂ |
| NickFect 36 | Stearyl- AG**Y**LLGK(K-Asp, Glu)**I**NLKALAALAKKIL-NH₂ |
| NickFect 37 | Stearyl- AG**O**(K-Glu₂)LLGK**I**NLKALAALAKKIL-NH₂ |
| NickFect 38 | Stearyl- AG**O**(K-Asp₂)LLGK**I**NLKALAALAKKIL-NH₂ |
| NickFect 39 | Stearyl- AG**O**(K-Glu, Asp)LLGK**I**NLKALAALAKKIL-NH₂ |
| NickFect 41 | AG**K**(K-Glu₂)LLGK(Stearyl)**I**NLKALAALAKKIL-NH₂ |
| NickFect 42 | AG**K**(K-Asp₂)LLGK(Stearyl)**I**NLKALAALAKKIL-NH₂ |
| NickFect 43 | AG**K**(K-Asp, Glu)LLGK(Stearyl)**I**NLKALAALAKKIL-NH₂ |
| NickFect 51 | δ - (Stearyl-AGYLLG) **O**INLKALAALAKKIL-NH₂ |
| NickFect 52 | α, ε (Stearyl-AGYLLG)₂ **K** INLKALAALAKKIL-NH₂ |
| NickFect 53 | ε - (Steary- AGYLLG) **K** INLKALAALAKKIL-NH₂ |
| NickFect 54 | α-(RPPGFSPFR)-ε-(Stearyl-AGYLLG)-**K** INLKALAALAKKIL-NH₂ |
| NickFect 55 | α-(ALALLL)-ε-(Stearyl-AGYLLG)-**K** INLKALAALAKKIL-NH₂ |
| NickFect 56 | α-(RPKPQQFGLM)-δ-(Stearyl-AGYLLG ) **O** INLKALAALAKKIL-NH₂ |
| NickFect 64 | Stearyl- AGYLLG**O**INL**O**ALAALA**OO**IL-NH₂ |
| NickFect 71 | AGYLLG**O**(Stearyl)INLKALAALAKKIL-NH₂ |
| NickFect 72 | AGYLLG**O**(Stearyl)INL**O**ALAALAKKIL-NH₂ |
| NickFect 73 | AGYLLG**O**(Stearyl)INL**O**ALAALA**O**KIL-NH₂ |
| NickFect 73 | AGYLLG**O**(Stearyl)INL**O**ALAALA**OO**IL-NH₂ |
| NickFect 81 | Stearyl- AGYLLG**K**IN-Nle-KALAALAKKIL-NH₂ |
| NickFect 82 | Stearyl- AGYL-Nle-G**K**IN-Nle-KALAALAKKIL-NH₂ |
| NickFect 82 | Stearyl- AGYL-Nle-G**K**IN-Nle-KA-Nle-AALAKKIL-NH₂ |
| NickFect 91 | **K**(Asp₂)AGYLLG**K**(Stearyl)INLKALAALAKKIL-NH₂ |
| NickFect 92 | **K**(Glu₂)AGYLLG**K**(Stearyl)INLKALAALAKKIL-NH₂ |
| NickFect 93 | **K**(Asp, Glu)AGYLLG**K**(Stearyl)INLKALAALAKKIL-NH₂ |
| NickFect 94 | **K**(Asp, Stearyl)AGYLLG**K**INLKALAALAKKIL-NH₂ |
| NickFect 95 | **O**(Asp₂)AGYLLG**K**(Stearyl)INLKALAALAKKIL-NH₂ |

### Examples and Experiments

The invention below described by examples and comparisons with NickFect and PepFect systems and also with Lipofectamine 2000 as positive control, which is market leading transfection reagent for in vitro today. First the improvement and remodeling of the delivery vectors are described. Then the testing of various methods to illustrate how versatile NickFects are for delivery of splice correcting oligonucleotides, plasmids and siRNA is being described. In addition, the overall improvement of NickFect peptides compared to Lipofectamine 2000, which have been applied according to the manufacturer's protocol, is shown by lower cytotoxicity, homogenious and efficient transfections. All experiments have been performed in more than one cell type and in most cases with serum present.

### Materials and methods

### Synthesis of peptides

Peptides were synthesized in stepwise manner at 0.1 mmol scale on an automated peptide synthesizer (Applied Biosystems, ABI433A,USA) using Fmoc (fluorenylmethyloxycarbonyl) solid-phase peptide synthesis strategy (Fields and Noble, 1990) with Rink-amide MBHA (methylbenzylhydrylamine) resin (Fluka) as solid phase to obtain *C*-terminally amidated peptides. The stearic acid was coupled manually to the *N-*terminus of the peptide by treatment of peptidyl-resins with 5 eq. stearic acid (Sigma, Germany), 3 eq. HOBt and 3 eq. HBTU (MultiSyntech, Germany), 6 eq. DIEA (Sigma, Germany) in dimethylformamide/dichloromethane (1:1) overnight at room temperature. For synthesis of phosphorylated peptides phosphothreonine Fmoc-Thr(PO(OBzI)OH)-OH (Fluka, Germany) and phosphotyrosine Fmoc-Tyr(PO(OBzl)OH)-OH (Merck, Germany) monomers were used and the coupling was carried out manually by treatment of peptidylresin with 3 eq. of a phosphomonomer, 3 eq. HOBt and 3eq. HBTU, 6 eq. DIEA for 3 h at room temperature in DCM/DMF/NMP/DMSO (3:3:3:1; v:v:v:v) mixture to increase the yield. The final cleavage was performed using standard protocol (95% TFA/2.5% TIS/2.5% H₂O. Peptides were purified by reversed-phase HPLC using C18 column (Phenomenex Jupiter C4, 5µm, 300A, 250x10mm) using a gradient of 20-80% acetonitrile/water containing 0.1% TFA. The identity of peptides was analyzed by MALDI-TOF mass-spectroscopy (The Voyager-DE™ PRO Biospectrometry™ System) in positive linear mode using α-cyano-4-hydroxycinnamic acid as matrix (Sigma-Aldrich). The molarity of the peptides was determined based on dilutions of accurately weighed substances. The sequences of the peptides are presented in Table 1.

Cy5 labeled and unlabeled phosphorothioate 2'-O-methyl RNA oligonucleotides (CCU CUU ACC UCA GUU ACA) were purchased from Microsynth AG, Switzerland.

### Cell culture

HeLa pLuc 705 cells, kindly provided by R. Kole and B. Leblue, and HEK293 cells were grown at 37 °C, 5% CO₂ in Dulbecco's Modified Eagle's Media (DMEM) with glutamax supplemented with 0.1 mM non-essential amino acids, 1.0 mM sodium pyruvate, 10 % FBS, 100 U/ml penicillin, 100 mg/ml streptomycin. CHO cells were grown in DMEM-F12 media with glutamax supplemented with 0.1 mM non-essential amino acids, 1.0 mM sodium pyruvate, and 10 % FBS, 100 U/ml penicillin, and 100 mg/ml streptomycin. Cells were grown at 37 °C in 5% CO₂ atmosphere. All media and chemicals were purchased from PAA Laboratories GmbH (Germany).

### Complex formation

Formation of SCO/CPP complexes: Phosphorothioate 2 -OMe oligonucleotides were mixed with CPPs at different molar ratios (1:0-1:20) in ddH₂O in 10% of the final treatment volume (i.e. 50 µl). Complexes were formed for 1 h at room temperature and meanwhile the cell medium was replaced in 24-well plates to fresh serum free DMEM (450 µl). Thereafter complexes were added to each well. When using Lipofectamine™ 2000 (Invitrogen, USA), the complexes were prepared according to manufacturer's protocol in OPTIMEM medium (Invitrogen, USA).

Formation of plasmid/CPP complexes: 0.5 µg of pGL3 luciferase expressing plasmid or pEGFP green fluorescent protein expressing vector were mixed with CPPs at different charge ratios (1:1-1:5) in ddH₂O in 1/10^{th} of the final treatment volume (50 µl). After 1 hour, complexes were added to cells grown in 450 µl of fresh serum free media. When using Lipofectamine 2000, complexes were prepared according to manufacturers protocol (Promega, USA).

### Gel redardation assay

The formation of oligonucleotides/CPP complexes was analyzed using Cy5 labeled ON by Typhoon Variable Mode Imager (Amersham, Sweden) after electrophoresis of complexes in 2% agarose gel in Tris-acetate-EDTA (TAE) buffer for 30 min at 100V.

Plasmid/CPP complexes were analyzed electrophoresis on a 2% agarose gel in TAE buffer, containing ethidium bromide (Sigma, Germany), for 1 hour at 100V.

### Splice correction assay

Cells (50,000) were seeded 24 h prior to experiments in 24-well plates to reach about 60 % confluence one day post seeding. Cells were treated with peptide: 2'-OMe ON complexes at six different molar ratios (1:1, 3:1, 5:1, 7:1, 10:1, 15:1) at 200nM oligonucleotide concentration for 4 h in 500 µl serum-free or serum-containing media, followed by the addition of 1 ml 10% serum-containing medium and incubated for additional 20 h. Thereafter, the cells were washed with PBS buffer and lysed using 100 µl 0.1% Triton X-100 in HEPES-Krebs-Ringer (HKR) buffer for 30 min at 4 °C. Luciferase activity was measured using Promega's luciferase assay system on GLOMAX™ 96 microplate luminometer (Promega, Sweden) according to suggestion by manufacturer and normalized to protein content by using DC protein determination assay (Bio-Rad, USA) for protein concentration measurement. Lipofectamine™ 2000 was used as a positive control for measuring transfection efficiency and naked oligonucleotides were used as a negative control.

In experiments with sodium 1-naphthyl phosphate monohydrate - N7000 (final concentration 200 µM), inhibitor was added to the medium 30 min prior to treatment of cells with peptide: 2'-OMe ON complexes. 2 h after addition of the complexes to cells, medium was removed and replaced with fresh medium in order to avoid toxicity effects of inhibitors. In experiments with chloroquine, chloroquine (final concentration 100 µM) was added to cells along with peptide: 2'-OMe ON complexes in order to promote endosomal escape. 4 h after addition of the complexes and chloroquine to cells, medium was replaced with fresh medium in order to avoid toxic effects of chloroquine.

### Plasmid transfection

50 000 CHO (Chinese hamster ovary cell) or HEK293 (Human Embryonic Kidney 293 cells) cells were seeded 24 h before the experiment into 24-well plates. Cells were treated with plasmid:CPP complexes at different charge ratios (1:1, 1:2, 1:3, 1:5) for 4 h in serum-free or full media. Lipofectamine™ 2000 (Invitrogen, Sweden) was used according to the manufacturer's protocol.

After 4 h treatment 500 µl of full growth media was added and incubated for another 20 h. Thereafter, the cells were washed twice with HKR and lysed using 100 µl 0.2% Triton X-100 in HKR buffer for 30 min at room temperature. In case of pGL3 plasmid luciferase activity was measured using Promega's luciferase assay system on GLOMAX™ 96 microplate luminometer (Promega, Sweden) and in case of pEGFP vector fluoresceines measurement was done on Biotek Synergy Mx monochromator-based multi-mode microplate reader (BioTek Instruments, Inc., USA). Data was normalized to protein content measured using DC protein determination kit (Bio-Rad Laboratories, Inc, USA)

### FACS (siRNA downregulation)

EGFP-CHO cells (10 000) we seeded 24 h before experiment in 96-wellplate to reach 60% confluence on the day of the experiment. For complex formation peptide (100 µM stock solution) was mixed with siRNA (10 µM stock solution) in MQ water in 1/10th of final treatment volume (i.e. 10 µl). Final concentrations: 100 nM siRNA, MR 20, MR25, MR30, MR40 in serum free DMEM were used.

Complexes were formed for 60 min at room temperature and added to cells in 100 µl growth media. After 4 h, 100 µl of fresh media was added to wells and cells were incubated for additional 20 h. Treatments with LF2000 were made in accordance with recommendations from manufacturer (Invitrogen) After 24 h, media was removed. Cells were rinsed with PBS and detached from the plate using Trypsin/EDTA in PBS for min. at 37° C. Cells were suspended with PBS containing 5% fetal bovine serum (FBS).

Flow cytometry analysis was carried out with BD FacsCanto flow cytometer (BD Biosciences, San Jose, CA, USA). Population of viable cells was determined from a scatter plot: forward scattered light (FSC) vs. side scattered liht (SSC) plot. A minimum 10,000 events from the viable cell population per sample were analyzed.

### Toxicity measurements

Cell proliferation was studied with CellTiter 96® AQueous Non-Radioactive Cell Proliferation Assay (MTS) according to the manufacturer's instructions. Briefly, 10000 Hela pLuc 705 cells per well were seeded 1 day prior to experiment on a 96-well plate in complete DMEM. Cells were treated with peptide: 2'-OMe ON complexes at four different molar ratios (5:1, 7:1, 10:1 and 20:1) for 4 hours in serum-free medium, followed by the addition of 10% serum containing medium and incubated for additional 20 hours. MTS was added according to the manufacturer's general protocol (Promega Biotech AB, Sweden). The conversion of MTS into aqueous, soluble formazan is accomplished by dehydrogenase enzymes found in metabolically active cells. The absorbance of formazan product was measured at 490 nm, which is directly proportional to the number of living cells in culture. Absorbance was measured on Tecan Sunrise microplate absorbance reader

(Tecan Group Ltd., Switzerland) and the percentage of viable cells was determined using GraphPad Prism software 5.0 (Graphpad Software , CA, USA).

### References

1. Cross, D. & Burmester, J.K. Gene therapy for cancer treatment: past, present and future. Clin Med Res 4, 218-27 (2006).
2. Kim, D.H. & Rossi, J.J. Strategies for silencing human disease using RNA interference. Nat Rev Genet 8, 173-84 (2007).
3. Mercatante, D.R., Sazani, P. & Kole, R. Modification of alternative splicing by antisense oligonucleotides as a potential chemotherapy for cancer and other diseases. Curr Cancer Drug Targets 1, 211-30 (2001).
4. EL-Andaloussi, S., Holm, T. & Langel, U. Cell-penetrating peptides: mechanisms and applications. Curr Pharm Des 11, 3597-611 (2005).
5. Mae, M. & Langel, U. Cell-penetrating peptides as vectors for peptide, protein and oligonucleotide delivery. Curr Opin Pharmacol 6, 509-14 (2006).
6. El-Andaloussi, S., Jarver, P., Johansson, H.J. & Langel, U. Cargo-dependent cytotoxicity and delivery efficacy of cell-penetrating peptides: a comparative study. Biochem J 407, 285-92 (2007).
7. Abes, S. et al. Vectorization of morpholino oligomers by the (R-Ahx-R)4 peptide allows efficient splicing correction in the absence of endosomolytic agents. J Control Release 116, 304-13 (2006).
8. Bendifallah, N. et al. Evaluation of cell-penetrating peptides (CPPs) as vehicles for intracellular delivery of antisense peptide nucleic acid (PNA). Bioconjug Chem 17, 750-8 (2006).
9. Lundberg, P., El-Andaloussi, S., Sutlu, T., Johansson, H. & Langel, U. Delivery of short interfering RNA using endosomolytic cell-penetrating peptides. Faseb J 21, 2664-71 (2007).
10. Derossi, D., Joliot, A.H., Chassaing, G. & Prochiantz, A. The third helix of the Antennapedia homeodomain translocates through biological membranes. J Biol Chem 269, 10444-50 (1994).
11. Soomets, U. et al. Deletion analogues of transportan. Biochim Biophys Acta 1467, 165-76 (2000).
12. El-Andaloussi, S., Johansson, H.J., Holm, T. & Langel, U. A Novel Cell-penetrating Peptide, M918, for Efficient Delivery of Proteins and Peptide Nucleic Acids. Mol Ther 15, 1820-6 (2007).
13. Wender, P.A. et al. The design, synthesis, and evaluation of molecules that enable or enhance cellular uptake: peptoid molecular transporters. Proc Natl Acad Sci U S A 97, 13003-8 (2000).
14. Kang, S.H., Cho, M.J. & Kole, R. Up-regulation of luciferase gene expression with antisense oligonucleotides: implications and applications in functional assay development. Biochemistry 37, 6235-9 (1998).
15. Morris, M.C., Vidal, P., Chaloin, L., Heitz, F. & Divita, G. A new peptide vector for efficient delivery of oligonucleotides into mammalian cells. Nucleic Acids Res 25, 2730-6 (1997).
16. Morris, M.C., Chaloin, L., Mery, J., Heitz, F. & Divita, G. A novel potent strategy for gene delivery using a single peptide vector as a carrier. Nucleic Acids Res 27, 3510-7 (1999).
17. Du, L., Pollard, J.M. & Gatti, R.A. Correction of prototypic ATM splicing mutations and aberrant ATM function with antisense morpholino oligonucleotides. Proc Natl Acad Sci U S A 104, 6007-12 (2007).
18. Garcia-Blanco, M.A., Baraniak, A.P. & Lasda, E.L. Alternative splicing in disease and therapy. Nat Biotechnol 22, 535-46 (2004).

## Claims

1. A system for cargo delivery into the cells comprising of cell penetrating peptide B which may comprise covalently linked component A and covalently or non-covalently linked peptide or non-peptide construct C, as targeting moiety for delivering cargo into cells.

2. The system according to claim 1, wherein peptide B comprises chemical modifications of cell penetrating peptide TP10 which has any fatty acid (e.g. stearic acid) covalently linked to the backbone .

3. The system according to claims 1-2, where Tyr in position 3 is replaced by Lys, Orn, Thr, Ser, Asp or Glu.

4. The system according to claims1-3, where Ile in position 8 is replaced by Lys, Orn, Thr, Ser, Tyr, Asp, Glu or any other hydrophilic amino acid.

5. The system according to any claims 1-4, where at least one Leu is replaced by leucine's isomers and/or analogues (e.g. Norleucine).

6. The system according to any claims1-5, where at least one lysines are replaced by lysine's isomers and/or analogues (e.g.Ornithine).

7. The system according to any claims 1-6, wherein peptide B is a branched peptide comprising of fatty acid modified TP10 peptide or/and its segments (e.g. Galanin, Mastoparan) linked through α, β, γ, δ, ε-amino groups of lysine or their analogues to amphipatic or/and α-helical peptides or/and peptide segments (e.g. NPY, substance P, bradykinin, model sequences like (Ala-Leu)ₙ, TP10, Galanin, Mastoparan)

8. The delivery system according to any claims 1-7, wherein one or more components A are conjugated to peptide B directly or via spacer.

9. The delivery system according to any claims 1-8, wherein linked components A may different.

10. The system according to any claims 1-9, wherein component A comprises phosphate group (PO₃) or any negatively charged moiety (selected from the group consisting of but not limited Asp, Glu, carbohydrates) or can be even a peptide sequence with overall negative charge.

11. The system according to any claims 1-10 comprising at least one component C, which is a targeting moiety

12. The system according to claim 11, wherein the targeting moiety is a cell- or tumor homing peptide, aptamer, a receptor ligand, a spacer comprising a cleavable site coupled to an inactivating peptide, peptide ligand, cytotoxic peptide, bioactive peptide ligand for a known or unknown receptor, a peptide sequence which selectively binds to a certain tissue or cell type or nuclear localization sequence (NLS).

13. The system according to any claims 1-12, further comprising a cargo, where one or several cargoes are linked covalently or/and non-covalently to component B.

14. The system according to any claims 1-13, wherein one or more components A, one or more components C and one or more cargoes are attached to peptide B.

15. The system according to any claims 1-14, comprising more than one peptide B.

16. The system according to any claims 1-15, wherein at least one of component C and cargo is attached with spacer arm.

17. The system according to any claims 1-16, wherein the cargo is selected from the group consisting of but not limited oligonucleotides and modified versions thereof, including single strand oligonucleotides (DNA, RNA, PNA, LNA and their analogues), double-strand oligonucleotides (siRNA, shRNA, decoyDNA), plasmids and other varieties thereof synthetic nucleotide analogues.

18. The system according to any claims 1-16, wherein the cargo is selected from the group consisting of a cell- or tumor homing peptide, aptamer, a receptor ligand, a spacer comprising a cleavable site coupled to an inactivating peptide, peptide ligand, cytotoxic peptide, bioactive peptide, antibody, diagnostic agent, protein, pharmaceutical e.g. anticancer drug or antibiotics.

19. The system according to any claims 1-17, further comprising at least one imaging agent and/or labelling molecule.

20. The system according to any claims 1-19, comprising in conjugation circulation clearance modifiers, like PEG.

21. The system according to any claims 1-20, wherein the total surface charge of formed peptide B and cargo nanoparticle is negative.

22. A composition comprising more than one delivery system according to any of claims 1-21.

23. Use of the system according to any claims 1-22 in diagnosis of diseases, as research tool, as targeting system and as a pharmaceutical composition.
